# FASCICULE DE BREVET EUROPEEN

(11) **EP 0 473 508 B1**
(45) Date de publication et mention de la délivrance du brevet: **17.08.1994**
(21) Numéro de dépôt: 91402320.5
(22) Date de dépôt: 28.08.1991
(51) Int. Cl.: A61K 7/06, A61K 7/48, A61K 7/50

(54) **Compositions de lavage à base de silicones et procédé de mise en oeuvre**
Silicone enthaltende Reinigungsmittel und Verfahren zu deren Verwendung
Cleansing composition containing silicones and method of use

(30) Priorité: 31.08.1990 FR 9010894
(43) Date de publication de la demande: 04.03.1992
(73) Titulaire: L'OREAL, 75008 Paris (FR)
(72) Inventeur: Beauquey, Bernard, F-92110 Clichy (FR); Dubief, Claude, F-78150 Le Chesnay (FR)
(74) Mandataire: Casalonga, Axel

(56) Documents cités:
- EP-A- 0 141 593
- EP-A- 0 158 174
- EP-A- 0 323 594
- US-A- 4 559 227

## Description

La présente invention est relative à des compositions de lavage et de conditionnement des matières kératiniques, en particulier des cheveux et/ou de la peau, et comportant dans un milieu aqueux, au moins une silicone insoluble en présence d'agents tensio-actifs, ainsi qu'aux procédés de lavage mettant en oeuvre ces compositions.

Les compositions de lavage des matières kératiniques, notamment les shampooings, sont bien connues dans l'état de la technique.

On a déjà proposé d'utiliser des silicones dans de telles compositions, notamment des silicones insolubles conférant aux matières kératiniques traitées, et en particulier aux cheveux, des propriétés intéressantes de conditionnement telles que douceur de brillance et de démêlage.

Compte tenu du caractère insoluble de ces silicones dans les milieux aqueux habituellement utilisés dans les shampooings, on cherche à maintenir les silicones sors forme dispersée tout en conservant les propriétés détergentes et moussantes de la composition et sans faire chuter la viscosité. Ces compositions doivent également conférer aux cheveux les propriétés de douceur, de brillance et de démêlage recherchées.

La demanderesse a découvert que, de façon surprenante, il était possible de préparer des compositions de lavage, particulièrement stables dans le temps et possédant de bonnes propriétés de détergence tout en conférant aux cheveux de la brillance, de la douceur et une facilité de peignage, en utilisant, en plus des agents tensio-actifs détergents, un mélange de deux agents tensio-actifs choisis parmi (a) un myristate d'un alcool polyhydrique en C₂-C₄, et (b) un alkyl-éther d'un alcanol(C₁-C₄)amide et/ou un alcanol(C₁-C₄) amide dérivé d'un acide gras en C₈-C₁₆.

La demanderesse a constaté en particulier que ces deux agents tensio-actifs, lorsqu'ils sont utilisés seuls en présence d'un agent tensio-actif détergent et d'une silicone insoluble, ne permettent pas d'obtenir des compositions stables sous forme de suspension, mais que leur association permet à la fois de résoudre le problème de stabilité dans le temps des compositions, de véhiculer la silicone sur les cheveux et de conférer à ceux-ci de bonnes propriétés de douceur, de démêlage et de brillance. Ces compositions possèdent également d'excellentes propriétés moussantes et lavantes sans être difficiles à rincer.

Ces compositions présentent, par ailleurs, l'avantage de pouvoir être réalisées sans difficulté à l'aide des procédés et des matériels classiquement utilisés.

L'invention a donc pour objet une composition lavante contenant au moins une silicone insoluble, un agent tensio-actif détergent et l'association de deux agents tensio-actifs particuliers définis ci-après.

Un autre objet de l'invention est constitué par le procédé de lavage mettant en oeuvre ces compositions.

D'autres objets de l'invention apparaîtront à la lecture de la description et des exemples qui suivent.

La composition conforme à l'invention est essentiellement caractérisée par le fait qu'elle contient dans un milieu aqueux
- au moins un agent tensio-actif possédant des propriétés détergentes,
- au moins une silicone insoluble,
- au moins un myristate d'un alcool polyhydrique en C₂-C₄,
- au moins un alkyléther d'un alcanol(C₁-C₄) amide et/ou un alcanol(C₁-C₄)amide dérivé d'acide gras en C₈-C₁₆.

Les différents composants de la composition de lavage conforme à l'invention sont utilisés de préférence dans les proportions suivantes, ces proportions étant exprimées en poids du poids total de la composition.
- 5 à 50% de l'agent tensio-actif possédant des propriétés détergentes,
- 0,1 à 20% d'au moins une silicone insoluble,
- 1 à 8% de myristate d'alcool polyhydrique en C₂-C₄,
- 0,5 à 8% d'alkyléther d'alcanolamide et/ou d'alcanol(C₁-C₄)amide d'acide gras en C₈-C₁₆.

Les agents tensio-actifs détergents sont choisis parmi les agents tensio-actifs anioniques, nonioniques, amphotères, zwittérioniques ou leurs mélanges.

Parmi les agents tensio-actifs anioniques, on peut citer plus particulièrement les acyliséthionates, les alkyl méthyl taurates, les sels alcalins, de magnésium, d'ammonium, d'amines or d'amino alcools des composés suivants :
- les alkylsulfates, les alkyléthersulfates, les alkylamidoéthersulfates, les alkylarylpolyéthersulfates, les monoglycérides sulfates;
- les alkylsulfonates, les alkylamides sulfonates, les alkylarylsulfonates, les oléfines sulfonates, les paraffines sulfonates;
- les alkylsulfosuccinates, les alkyléther sulfosuccinates, les alkylamide-sulfosuccinates;
- les alkylsulfosuccinamates;
- les alkylsulfoacétates;
- les alkylphosphates, les alkyléther phosphates;
- les acylsarcosinates.

Le radical alkyle ou acyle de ces différents composés sont généralement constitués par une chaîne carbonée comportant de 12 à 20 atomes de carbone.

Parmi les agents tensio-actifs anioniques, on peut également citer :

Les sels d'acides gras tels que les sels des acides oléique, ricinoléique, palmitique, stéarique; des acides d'huile de coprah ou d'huile de coprah hydrogénée; les acyl lactylates, dont le radical acyle comporte de 8 à 20 atomes de carbone.

Parmi les agents tensio-actifs considérés comme faiblement anioniques, on peut citer :

Les acides éthers carboxyliques polyoxy-alkylénés répondant à la formule :

R-(OC₃H₆)ₚ-(OC₂H₄)ₙ-OCH₂COOA

dans laquelle R désigne un radical alkyle ou un mélange de radicaux alkyle ou alcényle en C₈-C₂₂, linéaire ou ramifié, un radical alkyl(C₈-C₉)phényle, R′CONH-CH₂-CH₂- avec R′ désignant un radical alkyle ou alcényle, linéaire ou ramifié, en C₁₁-C₂₁;
n est un nombre entier ou décimal compris entre 2 et 24,
p est un nombre entier ou décimal compris entre 0 et 6,
A désigne un atome d'hydrogène ou bien Na, K, Li, 1/2 Mg ou un reste monoéthanolamine, ammonium ou triéthanolamine.

Les agents tensio-actifs non-ioniques sont plus particulièrement choisis parmi les alcools, les alkylphénols, les acides gras polyéthoxylés, poly propoxylés ou polyglycérolés à chaîne grasse comportant 8 à 18 atomes de carbone. Le nombre de groupements oxyde d'éthylène, oxyde de propylène étant compris entre 2 et 50 et le nombre de groupements glycérol étant compris entre 2 et 30.

On peut également citer les copolymères d'oxydes d'éthylène et de propylène, des condensats d'oxydes d'éthylène et de propylène sur des alcools gras, les amides gras polyéthoxylés ayant de préférence 2 à 30 moles d'oxyde d'éthylène, les amides gras poly-glycérolés comportant 1 à 5 groupements glycérol et de préférence 1,5 à 4, les amines grasses polyéthoxylées, ayant de préférence 2 à 30 moles d'oxyde d'éthylène, les esters d'acides gras du sorbitan oxyéthylénés, ayant de préférence 2 à 30 moles d'oxyde d'éthylène, les esters d'acides gras de sucroses, les esters d'acides gras du polyéthylèneglycol, les triesters phosphoriques, des oxydes d'amines tels que les oxydes d'alkylamines or de N-acylamidopropylmorpholine.

Les agents tensio-actifs amphotères ou zwittérioniques plus particulièrement préférés sont :
- les dérivés d'amines secondaires ou tertiaires aliphatiques dans lesquels le radical aliphatique est une chaîne linéaire ou ramifiée comportant 8 à 18 atomes de carbone et qui contient au moins un groupe anionique hydrosolubilisant carboxylique, sulfonate, sulfate, phosphate ou phosphonate;
- les alkyl(C₈-C₂₀)bétaïnes, les sulfobétaïnes, les alkyl(C₈-C₂₀)amido alkyl(C₁-C₆)bétaïnes ou les alkyl(C₈-C₂₀)amido alkyl(C₁-C₆)sulfobétaïnes.

Parmi ces composés, on peut citer les produits vendus sous la dénomination "MIRANOL", tels que décrits dans des brevets US-A-2 528 378 et 2 781 354 et classés dans le dictionnaire CTFA, 3ème édition, 1982, sous la dénomination d'Amphocarboxyglycinates et Amphocarboxypropionates.

Les alkylbétaïnes sont choisies de préférence parmi les alkyl(C₁₀-C₂₀)bétaïnes.

Les silicones utilisées conformément à l'invention sont des polyorganosiloxanes insolubles dans le milieu aqueux et peuvent se présenter sous forme d'huiles, de cires, de résines ou de gommes.

On utilise de préférence des silicones non volatiles et plus particulièrement des polyalkylsiloxanes, des polyarylsiloxanes, des polyalkylarylsiloxanes, des gommes et des résines de silicones, des polyorganosiloxanes modifiés par des groupements organofonctionnels ainsi que leurs mélanges.

Ces silicones sont plus particulièrement choisies parmi les polyalkylsiloxanes parmi lesquels on peut citer les polydiméthylsiloxanes linéaires à groupements terminaux triméthylsilyle ayant une viscosité de 5.10⁻⁶ à 2,5 m²/s à 25°C et de préférence 1.10⁻⁵ à 1 m²/s.

Parmi ces polyalkylsiloxanes, on peut citer à titre non limitatif les prodiits commerciaux suivants :
les huiles SILBIONE des séries 47 et 70 047 commercialisées par RHONE POULENC telles que les huiles 47 V 500 000 et 47 V 300;
les huiles de la série 200 de la Société DOW CORNING;
les huiles VISCASIL de GENERAL ELECTRIC et certaines huiles des séries SF (SF 96, SF 18) de GENERAL ELECTRIC.

On peut également citer les polydiméthyl siloxanes linéaires à groupements terminaux diméthyl silanol tels que les huiles de la série 48 de la Société RHONE POULENC.

Dans cette classe de polyalkylsiloxanes, on peut également citer les produits vendus sors les dénominations "ABIL WAX 9800 et 9801" par la Société GOLDSCHMIDT qui sont des polyalkyl (C₁-C₂₀) siloxanes.

Les polyalkylarylsiloxanes sont choisis parmi les polydiméthyl méthylphénylsiloxanes, les polydiméthyldiphénylsiloxanes linéaires et/ou ramifiés, de viscosité de 1.10⁻⁵ à 5.10⁻² m²/s à 25°C.

Parmi ces polyalkylarylsiloxanes, on peut citer à titre d'exemple les produits commercialisés sous les dénominations suivantes :
. les huiles SILBIONE de la série 70 641 de RHONE POULENC;
. les huiles des séries RHODORSIL 70 633 et 763 de RHONE POULENC;
. l'huile DC 556 COSMETIC GRAD FLUID de DOW CORNING;
. les silicones de la série PK de BAYER comme le produit PK20;
. les silicones des séries PN, PH de BAYER comme les produits PN 1000 et PH 1000;
. certaines huiles des séries SF de GENERAL ELECTRIC, telles que SF 1023, SF 1154, SF 1250, SF 1265.

Les gommes de silicone utilisables conformément à l'invention, sont notamment des polydiorganosiloxanes ayant des masses moléculaires élevées comprises entre 200 000 et 1 000 000, utilisés seuls ou en mélange dans un solvant. Ce solvant peut être choisi parmi les silicones volatiles, les huiles polydiméthylsiloxanes (PDMS), les huiles polyphényl méthylsiloxanes (PPMS), les isoparaffines, le chlorure de méthylène, le pentane, le dodécane, le tridécane, le tétradécane ou leurs mélanges.

On peut plus particulièrement citer les produits suivants :
- les gommes polydiméthylsiloxane/méthyl vinylsiloxane,
- polydiméthylsiloxane/diphénylsiloxane,
- polydiméthylsiloxane/phénylméthylsiloxane,
- polydiméthylsiloxane/diphénylsiloxane/méthyl vinylsiloxane.

D'autres produits utilisables conformément à l'invention, sont des mélanges tels que :
. les mélanges formés à partir d'un poly diméthylsiloxane hydroxylé en bout de chaîne (dénommé diméthiconol selon la nomenclature du dictionnaire CTFA) et d'un polydiméthylsiloxane cyclique (dénommé cyclo méthicone selon la nomenclature du dictionnaire CTFA) tel que le prodrit Q2 1401 vendu par la Société DOW CORNING;
. les mélanges formés à partir d'une gomme polydiméthylsiloxane avec une silicone cyclique tel que le produit SF 1214 Silicone Fluid de la Société GENERAL ELECTRIC, ce produit est une gomme SE 30 correspondant à une diméthicone, ayant un poids moléculaire de 500.000 solubilisée dans l'huile SF 1202 Silicone Fluid correspondant au décaméthylcyclopentasiloxane;
. les mélanges de deux PDMS de viscosités différentes, et plus particulièrement d'une gomme PDMS et d'une huile PDMS, tels que les produits SF 1236 ou CF 1241 de la Société GENERAL ELECTRIC. Le produit SF 1236 est le mélange d'une gomme SE 30 définie ci-dessus ayant une viscosité de 20 m²/s et d'une huile SF 96 d'une viscosité de 5.10⁻⁶ m²/s. Ce produit comporte de préférence 15% de gomme SE 30 et 85% d'une huile SF 96. Le produit CF 1241 est le mélange d'une gomme SE 30 (33%) et d'une huile PDMS (67%) d'une viscosité de 10⁻³ m²/s.

Les résines d'organopolysiloxanes utilisables conformément à l'invention, sont des systèmes siloxaniques réticulés renfermant les unités R₂SiO₂_{/2}, RSiO₃_{/2} et SiO₄_{/2}.

Parmi ces résines, celles dans lesquelles R désigne un radical alkyle inférieur en C₁-C₆ ou un radical phényle, sont préférées.

On peut citer parmi ces résines le produit vendu sors la dénomination "DOW CORNING 593" ou ceux vendus sous les dénominations "SILICONE FLUID SS 4230 et SS 4267" par la Société GENERAL ELECTRIC et qui sont des siloxanes de structure diméthyl/triméthylsiloxane.

Les silicones organo modifiées utilisables conformément à l'invention, sont des silicones telles que définies précédemment et comportant dans leur structure un ou plusieurs groupements organofonctionnels directement fixé(s) sur la chaîne siloxanique or fixé(s) par l'intermédiaire d'un radical hydrocarboné.

Parmi ces silicones, on peut citer les silicones comportant :
. des groupements polyéthylèneoxy et/ou poly propylèneoxy comportant éventuellement des groupements alkyle tels que le produit dénommé diméthicone copolyol vendu par la Société DOW CORNING sous la dénomination DC 1248 et l'alkyl (C₁₂) méthicone copolyol vendue par la Société DOW CORNING sous la dénomination Q2 5200; les huiles SILWET L 722, L 7500, L 77, L 711 de la Société UNION CARBIDE;
. des groupements aminés substitués ou non, comme les produits vendus sous la dénomination GP4 Silicone Fluid et GP 7100 par la Société GENESEE ou les produits vendus sous les dénominations Q2 8220 et DC 929 par la Société DOW CORNING. Les groupements aminés substitués sont en particulier des groupements amino alkyle en C₁-C₄;
. des groupements thiols comme les produits vendus sors les dénominations "GP 72 A" et "GP 71" de GENESEE;
. des groupements carboxylates comme les produits décrits dans le brevet EP 186 507 de la Société CHISSO CORPORATION;
. des groupements alcoxylés comme le produit vendu sous la dénomination " SILICONE COPOLYMER F-755 " par SWS SILICONES et ABIL WAX 2428, 2434 et 2440 par la Société GOLDSCHMIDT;
. des groupements hydroxylés comme les poly organosiloxanes à fonction hydroxyalkyle décrits dans la demande de brevet français FR-A-85 16334, répondant à la formule (I) : 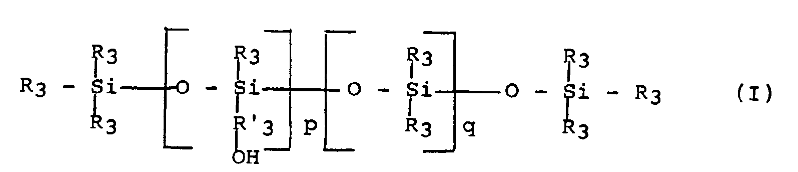 dans laquelle les radicaux R₃, identiques or différents, sont choisis parmi les radicaux méthyle et phényle, au moins 60% en mole des radicaux R₃ désignant méthyle; le radical R′₃ est un chaînon alkylène divalent hydrocarboné en C₂-C₁₈; p est compris entre 1 et 30 inclus; q est compris entre 1 et 150 inclus;
. des groupements acyloxyalkyle tels que par exemple les polyorganosiloxanes décrits dans la demande de brevet français FR-A-2.641.185 et répondant à la formule (II) : 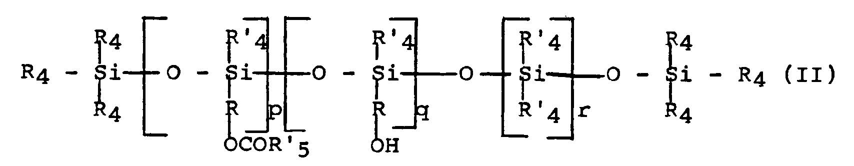 dans laquelle :
   R₄ désigne un groupement méthyle, phényle, -OCOR˝, hydroxyle, un seul des radicaux R₄ par atome de silicium pouvant être OH;
   R′₄ désigne méthyle, phényle, au moins 60% en proportion molaire de l'ensemble des radicaux R₄ et R′₄ désignant méthyle;
   R′₅ désigne alkyle or alcényle en C₈-C₂₀;
   R désigne un radical alkylène hydrocarboné divalent, linéaire or ramifié en C₂-C₁₈;
   r est compris entre 1 et 120 inclus;
   p est compris entre 1 et 30;
   q est égal à 0 ou est inférieur à 0,5 p, p+q étant compris entre 1 et 30; les polyorganosiloxanes de formule (II) peuvent contenir des groupements 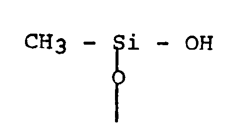 dans des proportions ne dépassant pas 15% de la somme p+q+r;
. des groupements anioniques de type carboxylique, tels que les groupements alkylcarboxyliques comme ceux présents dans le produit X-22-3701E de la Société SHIN-ETSU; de type 2-hydroxyalkylsulfonate; de type 2-hydroxyalkylthiosulfate, tels que les produits vendus par la Société GOLDSCHMIDT sous les dénominations "ABIL S201" et "ABIL S 255".
. des groupements hydroxyacylamino comme les produits décrits dans la demande EP-A-O 342 834. De telles silicones sont vendues en particulier sous la dénomination Q2-8413 par la Société DOW-CORNING.

On peut également utiliser des silicones possédant un point d'ébullition compris entre 60°C et 260°C.

Les silicones volatiles sont plus particulièrement choisies parmi :
(i) les silicones cycliques comportant de 3 à 7 atomes de silicium et de préférence 4 à 5. Il s'agit, par exemple, de l'octaméthylcyclotétrasiloxane vendu sous le nom de VOLATILE SILICONE 7207 par UNION CARBIDE ou SILBIONE 70045 V 2 par RHONE POULENC, le décaméthylcyclopentasiloxane vendu sous le nom de VOLATILE SILICONE 7158 par UNION CARBIDE, SILBIONE 70045 V 5 par RHONE POULENC, ainsi que leurs mélanges.
   On cite également les cyclocopolymères du type diméthylsiloxane/méthylalkylsiloxane, tel que la SILICONE VOLATILE FZ 3109 vendue par la Société UNION CARBIDE, dans laquelle alkyl représente C₈H₁₇.
   On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1′-(hexa-2,2,2′,2′,3,3′-triméthylsilyloxy)bis-néopentane;
(ii) les silicones volatiles linéaires ayant 2 à 9 atomes de silicium et possédant une viscosité inférieure ou égale à 5.10⁻⁶ m²/s à 25°C. Il s'agit, par exemple, de l'hexaméthyldisiloxane vendu sous la dénomination SILBIONE 70 041 V 0,65 par la Société RHONE POULENC et du décaméthyltétrasiloxane vendu sous la dénomination SH 200 par la Société TORAY SILICONE. Des silicones entrant dans cette classe sont également décrites dans l'article publié dans Cosmetics and toiletries, Vol. 91, Jan. 76, p. 27-32 - TODD & BYERS "Volatile Silicone fluids for cosmetics".

De façon préférentielle, la composition lavante selon l'invention contient :
- de 1,5 à 5% de myristate d'un alcool polyhydrique en C₂-C₄, constitué notamment par l'éthylèneglycol, le propylèneglycol et le glycérol.

Le myristate de l'alcool polyhydrique est un produit commercial qui est constitué au minimum de 95% de C₁₄.

Le dimyristate d'éthylèneglycol est particulièrement préféré.
- de 0,5 à 8% d'un amide choisi parmi :
   a) des alkyl éther d'alcanol(C₁-C₄)amides, la chaîne alkyle ayant de 13 à 15 atomes de carbone; le composé préféré est l'alcoxy (C₁₃-C₁₅) diéthoxyméthylmono éthanolamide tel que le produit vendu sous la dénomination AMINOL A15 par la Société CHEM'Y;
   b) des alcanol(C₁-C₄)amides d'acides gras en C₈-C₁₆ tels que le diéthanolamide de coprah, le monoéthanolamide de coprah ou le monoisopropanolamide de coprah;
- de 1 à 10% en poids de silicone et plus particulièrement de 1 à 4% en poids, par rapport au poids total de la composition.

Les agents tensio-actifs détergents sont dans des proportions suffisantes pour conférer un caractère détergent à la composition, en particulier comprises entre 8 et 30% en poids.

Dans les compositions conformes à l'invention, on utilise de préférence des mélanges d'agents tensio-actifs et en particulier des mélanges d'agents tensio-actifs anioniques et d'agents tensio-actifs amphotères, zwittérioniques ou non-ioniques. Un mélange particulièrement préféré est un mélange constitué d'un agent tensio-actif anionique et d'un agent tensio-actif zwittérionique.

On utilise de préférence un agent tensio-actif anionique choisi parmi les alkyl(C₁₂-C₁₄)sulfates de sodium, de triéthanolamine ou d'ammonium, les alkyl (C₁₂-C₁₄)éthersulfates de sodium oxyéthylénés à 2,2 moles d'oxyde d'éthylène, le cocoyliséthionate de sodium et les α-oléfine(C₁₄-C₁₆)sulfonates de sodium et leurs mélanges avec
- soit un agent tensio-actif amphotère tel qu'un amphocarboxyglycinate défini ci-dessus;
- soit un agent tensio-actif zwittérionique tel que la laurylbétaïne vendue sous la dénomination DEHYTON AB 30 en solution aqueuse à 32% de MA par la Société HENKEL.

Lorsque les agents tensio-actifs amphotères ou zwittérioniques sont utilisés en mélange avec des agents tensio-actifs anioniques, ils représentent jusqu'à 50% et de préférence de 5 à 30% en poids par rapport au poids total de la quantité d'agents tensio-actifs présents dans la composition.

Lorsque les agents tensio-actifs non-ioniques sont utilisés en mélange avec des agents tensio-actifs anioniques, ils représentent jusqu'à 90% et de préférence 5 à 50% en poids par rapport à la quantité totale d'agents tensio-actifs présents dans la composition.

Le pH de ces compositions est généralement compris entre 3 et 9 et plus particulièrement entre 4 et 8.

Selon une variante de l'invention, la composition contient :
- de 0,1 à 20% et de préférence de 0,1 à 10% de silicone insoluble;
- de 8 à 30% d'un agent tensio-actif détergent tel que défini ci-dessus;
- de 1 à 10% d'acyl(C₇-C₁₇)iséthionate ou d'alkyl méthyl taurate;
- de 1 à 8% de myristate d'alcool polyhydrique (C₂-C₄);
- de 0,5 à 8% d'alkyléther d'alcanolamide ou d'alcanolamide d'acides gras en C₈-C₁₆.

Le milieu aqueux peut être constitué uniquement par de l'eau ou un mélange d'eau et d'un solvant cosmétiquement acceptable, tel que des alcools inférieurs en C₁-C₄ comme l'éthanol, l'isopropanol, le n-butanol, les alkylèneglycols comme l'éthylèneglycol, les éthers de glycol.

Les compositions conformes à l'invention peuvent contenir en plus de l'association définie selon l'invention, des agents régulateurs de viscosité tels que des électrolytes, des hydrotopes ou d'autres épaississants. On peut citer parmi ces adjuvants, le chlorure de sodium, le xylène sulfonate de sodium.

Les agents régulateurs de viscosité sont utilisés, selon l'invention, dans des proportions pouvant aller jusqu'à 10% par rapport au poids total de la composition.

Les compositions conformes à l'invention peuvent également contenir jusqu'à 3% d'agents nacrants ou opacifiants, tels que les palmitates de sodium ou de potassium, les stéarates ou hydroxystéarates de sodium ou de potassium, le mono- ou distéarate d'éthylène glycol.

Les compositions conformes à l'invention peuvent également renfermer d'autres agents ayant pour effet d'améliorer les propriétés cosmétiques des cheveux ou de la peau, sans cependant altérer la stabilité des compositions, tels que des agents tensio-actifs cationiques, des polymères anioniques ou non-ioniques ou cationiques ou amphotères ou des protéines éventuellement quaternisées.

Ces compositions peuvent contenir également différents adjuvants habituellement utilisés en cosmétique, tels que des parfums, des colorants, des conservateurs, des séquestrants, des stabilisateurs de mousse et des agents acidifiants ou alcalinisants bien connus en cosmétique.

Les compositions selon l'invention sont utilisées de préférence comme shampooings pour le lavage et le conditionnement des cheveux et ils sont appliqués, dans ce cas-là, sur des cheveux humides ou secs dans des quantités efficaces pour les laver, cette application étant suivie d'un rinçage.

Les compositions conformes à l'invention sont également utilisables comme gels douche pour le lavage des cheveux et de la peau, auquel cas ils sont appliqués sur la peau et les cheveux humides et ils sont rincés après application.

Les exemples suivants sont destinés à illustrer l'invention.

### EXEMPLE 1

- Lauryl éther sulfate de sodium oxyéthyléné à 2,2 moles d'oxyde d'éthylène 11,2 g
- Lauryl bétaïne 2,4 g
- Isopropanolamide de coprah 4,0 g
- Dimyristate d'éthylèneglycol 3,0 g
- Polydiméthylsiloxane vendu sous la dénomination huile SILBIONE 700 47 V 500 000 par la Société RHONE POULENC 2,0 g
- Parfum, conservateurs, qs
- Eau qsp 100,0 g

Cette composition est utilisée pour laver les cheveux et la peau et confère aux cheveux après séchage, douceur, brillance et permet un bon démêlage et laisse la peau douce.

### EXEMPLE 2

- Lauryl éther sulfate de sodium oxyéthyléné à 2,2 moles d'oxyde d'éthylène 8,4 g
- Lauryl bétaïne 6,0 g
- Isopropanolamide de coprah 0,5 g
- Dimyristate d'éthylèneglycol 1,0 g
- Polydiméthylsiloxane vendu sous la dénomination huile SILBIONE 700 47 V 300 par la Société RHONE POULENC 0,5 g
- Parfum, conservateurs, qs
- Eau qsp 100,0 g

### EXEMPLE 3

- Alkyl(C₁₂-C₁₄)sulfate d'ammonium 7,5 g
- Lauryl éther sulfate d'ammonium (3 OE) 6,2 g
- Cocoyl iséthionate de sodium 6,0 g
- Lauryl bétaïne 1,2 g
- Alcoxy(C₁₃-C₁₅)diéthoxyméthylmonoéthanolamide 2,0 g
- Polydiméthylsiloxane (PM : 250 000 viscosité à 25°C : 500 000 cst) 3,0 g
- Chlorure de stéaryl diméthyl benzyl ammonium 1, 0 g
- Gomme d'hydroxypropyl guar quaternisée par du chlorure de 2,3-epoxypropyl triméthyl ammonium 0,15 g
- Dimyristate d'éthylèneglycol 2,5 g
- Eau déminéralisée stérilisée qsp 100,00 g

### EXEMPLE 4

- Alkyl(C₁₂-C₁₄)éther sulfate de sodium (2,2 OE) 14,0 g
- Cocoyl iséthionate de sodium 6,0 g
- Lauryl bétaïne 2,4 g
- Poly diméthylsiloxane (PM : 250 000 viscosité à 25°C : 500 000 cst) 3,0 g
- Dimyristate d'éthylèneglycol 2,5 g
- Alcoxy(C₁₃-C₁₅)diéthoxyméthyl mono éthanolamide 2,0 g
- Eau déminéralisée stérilisée qsp 100,00 g

Les compositions des exemples 2 à 4 confèrent aux cheveux et à la peau des propriétés similaires à celles mentionnées pour l'exemple 1.

## Revendications

1. Composition de lavage et de conditionnement des matières kératiniques, en particulier des cheveux et de la peau, caractérisée par le fait qu'elle contient dans un milieu aqueux
- au moins un agent tensio-actif détergent,
- au moins une silicone insoluble,
- au moins un myristate d'alcool polyhydrique en C₂-C₄,
- au moins un alkyléther d'alcanol(C₁-C₄) amide et/ou un alcanol (C₁-C₄) amide d'un acide gras en C₈-C₁₆.

2. Composition selon la revendication 1, caractérisée par le fait qu'elle contient :
- de 5 à 50% en poids d'au moins un agent tensio-actif détergent,
- de 0,1 à 20% en poids d'au moins une silicone insoluble,
- de 1 à 8% en poids de myristate d'alcool polyhydrique (C₂-C₄), et
- de 0,5 à 8% en poids d'alkyléther d'alcanol-amide et/ou d'alcanol(C₁-C₄)amide d'acide gras en C₈-C₁₆.

3. Composition selon la revendication 1 ou 2, caractérisée par le fait que les agents tensio-actifs détergents sont choisis parmi les agents tensio-actifs anioniques, amphotères ou zwittérioniques, non-ioniques ou leurs mélanges.

4. Composition selon l'une quelconque des revendications 1 à 3, caractérisée par le fait que les agents tensio-actifs anioniques sont choisis parmi les sels alcalins, les sels de magnésium, les sels d'ammonium, les sels d'amines ou les sels d'amino alcools des composés suivants :
- les alkylsulfates, les alkyléthersulfates, les alkylamidoéthersulfates, les alkylarylpolyéthersulfates, les monoglycérides sulfates;
- les alkylsulfonates, les alkylamides sulfonates, les alkylarylsulfonates, les oléfines sulfonates, les paraffines sulfonates;
- les alkylsulfosuccinates, les alkyléther sulfosuccinates, les alkylamide-sulfosuccinates;
- les alkylsulfosuccinamates;
- les alkylsulfoacétates;
- les alkylphosphates, les alkyléther phosphates;
- les acylsarcosinates;
le radical alkyle ou acyle de ces composés étant constitué d'une chaîne carbonée comportant 12 à 20 atomes de carbone;
- les sels d'acides gras choisis parmi les sels des acides oléique, ricinoléique, palmitique, stéarique; des acides d'huile de coprah ou d'huile de coprah hydrogénée; les acyl lactylates, dont le radical acyle comporte de 8 à 20 atomes de carbone;
- les acides d'éthers carboxyliques polyoxyalkylénés de formule :
R-(OC₃H₆)ₚ-(OC₂H₄)ₙ-OCH₂COOA
dans laquelle R désigne un radical alkyle ou un mélange de radicaux alkyle ou alcényle en C₈-C₂₂, linéaire ou ramifié, un radical alkyle(C₈-C₉)phényle, un groupement R′CONH-CH₂-CH₂- avec R′désignant un radical alkyle ou alcényle, linéaire ou ramifié en C₁₁-C₂₁;
n est un nombre entier ou décimal compris entre 2 et 24,
p est un nombre entier ou décimal compris entre 0 et 6,
A désigne un atome d'hydrogène ou bien Na, K, Li, 1/2 Mg ou un reste monoéthanolamine, ammonium ou triéthanolamine.

5. Composition selon l'une quelconque des revendications 1 à 4, caractérisée par le fait que l'agent tensio-actif est un agent tensio-actif nonionique choisi parmi :
- les alcools, les alkylphénols et les acides gras polyéthoxylés, polypropoxylés, polyglycérolés, à chaîne grasse linéaire comportant 8 à 18 atomes de carbone, le nombre des groupements oxyde d'éthylène, oxyde de propylène étant compris entre 2 et 50, et le nombre de groupements glycérol étant compris entre 2 et 30;
- les copolymères d'oxyde d'éthylène et de propylène;
- les condensats d'oxyde d'éthylène et de propylène sur des alcools gras;
- les amides gras polyéthoxylés;
- les amides gras polyglycérolés;
- les amines grasses polyéthoxylées;
- les esters d'acides gras du sorbitan oxyéthylénés;
- les esters d'acides gras de sucroses;
- les esters d'acides gras du polyéthylène glycol;
- les triesters phosphoriques;
- les oxydes d'amines.

6. Composition selon l'une quelconque des revendications 1 à 5, caractérisée par le fait que les agents tensio-actifs amphotères ou zwittérioniques sont choisis parmi :
- les dérivés d'amines secondaires ou tertiaires, aliphatiques, dans lesquels le radical aliphatique est une chaîne linéaire ou ramifiée comportant 8 à 18 atomes de carbone et qui contient au moins un groupe anionique hydrosolubilisant carboxylique, sulfonate, sulfate, phosphate ou phosphonate;
- les alkyl(C₈-C₂₀)bétaïnes, les alkyl(C₈-C₂₀) sulfobétaïnes, les alkyl(C₈-C₂₀)amidoalkyl(C₁-C₆) bétaïnes ou les alkyl(C₈-C₂₀)amidoalkyl(C₁-C₆)sulfobétaïnes.

7. Composition selon l'une quelconque des revendications 1 à 6, caractérisée par le fait que l'on utilise des mélanges d'agents tensio-actifs détergents et en particulier des mélanges d'agents tensio-actifs anioniques et d'agents tensio-actifs amphotères, zwittérioniques ou non-ioniques.

8. Composition selon l'une quelconque des revendications 1 à 7, caractérisée par le fait que la silicone est choisie parmi les polyorganosiloxanes insolubles dans les milieux aqueux, se présentant sous forme d'huiles, de cires, de gommes ou de résines.

9. Composition selon l'une quelconque des revendications 1 à 8, caractérisée par le fait que la silicone est une silicone non volatile choisie parmi les polyalkylsiloxanes, les polyarylsiloxanes, les polyalkylarylsiloxanes, les gommes et résines de silicones, les polysiloxanes organomodifiés, ainsi que leurs mélanges.

10. Composition selon la revendication 9, caractérisée par le fait que la silicone non volatile est choisie parmi :
- les polydiméthylsiloxanes linéaires à groupements terminaux triméthylsilyle,
- les polydiméthylsiloxanes linéaires à groupements terminaux diméthylsilanol,
- les polyalkyl(C₁-C₂₀)siloxanes comportant au moins une chaîne grasse en C₁-C₂₀,
- les polydiméthylméthylphénylsiloxanes, les polydiméthyldiphénylsiloxanes linéaires et/ou ramifiés,
- les gommes de silicones de masse moléculaire comprises entre 200.000 et 1.000.000, utilisées seules ou sous forme de mélanges dans un solvant,
- les résines d'organopolysiloxane constituées de systèmes siloxaniques réticulés renfermant les unités R₂SiO₂_{/2}, RSiO₃_{/2} et SiO₄_{/2}, dans lesquelles R représente un groupement hydrocarboné ayant de 1 à 6 atomes de carbone ou un groupement phényle;
- les silicones organomodifiées choisies parmi les silicones comportant dans leur structure, un ou plusieurs groupements organofonctionnels directement fixés sur la chaîne siloxanique ou fixés par l'intermédiaire d'un radical hydrocarboné.

11. Composition selon la revendication 10, caractérisée par le fait que les gommes de silicones sont choisies dans le groupe constitué par les copolymères suivants :
- poly/(diméthylsiloxane)/(méthylvinylsiloxane)/,
- poly/(diméthylsiloxane)/(diphénylsiloxane)/,
- poly/(diméthylsiloxane)/(phénylméthylsiloxane)/,
- poly/(diméthylsiloxane)/(diphénylsiloxane)/(méthylvinylsiloxane)/;
et les mélanges suivants :
- les mélanges formés à partir d'un polydiméthylsiloxane hydroxylé en bout de chaîne et d'un polydiméthylsiloxane cyclique;
- les mélanges formés à partir d'une gomme polydiméthylsiloxane et d'une silicone cyclique;
- les mélanges de deux polydiméthylsiloxanes de viscosités différentes.

12. Composition selon la revendication 10, caractérisée par le fait que les silicones organo-modifiées sont choisies parmi les polyorganosiloxanes comportant :
a) des groupements polyéthylèneoxy et/ou polypropylèneoxy, comportant éventuellement des groupements alkyle;
b) des groupements aminés, substitués ou non;
c) des groupements thiols;
d) des groupements carboxylates;
e) des groupements alcoxylés;
f) des groupements hydroxyalkyle, répondant à la formule suivante : 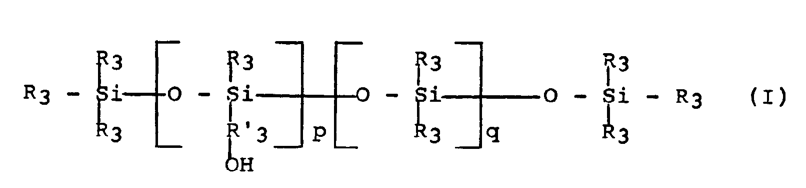 dans laquelle :
- les radicaux R₃, identiques ou différents, sont choisis parmi les radicaux méthyle et phényle, au moins 60% en moles des radicaux R₁ étant méthyle;
- le radical R′₃ est un chaînon alkylène divalent hydrocarboné en C₂-C₁₈;
- p est compris entre 1 et 30 inclus;
- q est compris entre 1 et 150 inclus.
g) des groupements acyloxyalkyle, répondant à la formule suivante : 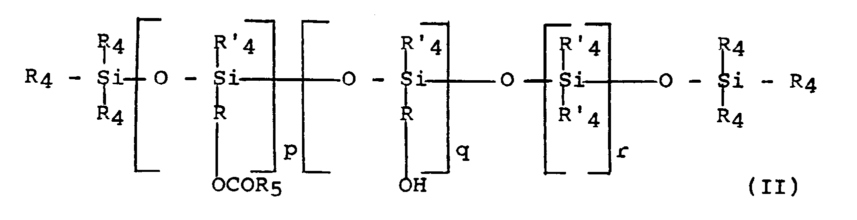 dans laquelle :
- R₄ désigne méthyle, phényle, -OCOR˝, hydroxyle, un seul des R₄ par atome de silicium peut être OH;
- R′₄ désigne méthyle, phényle, 60% molaire au moins de l'ensemble des radicaux R₄ et R′₄ est méthyle;
- R₅ désigne alkyle ou alcényle en C₈-C₂₀;
- R désigne un alkylène hydrocarboné divalent, linéaire ou ramifié, en C₂-C₁₈;
- r est compris entre 1 et 120 inclus;
- p est compris entre 1 et 30;
- q est égal à 0 ou est inférieur à 0,5 p, la somme p+q étant comprise entre 1 et 30; les polyorganosiloxanes de formule (II) pouvant contenir des groupements 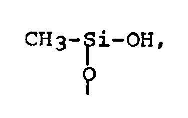 dans des proportions ne dépassant pas 15% de la somme p+q+r;
h) des groupements alkylcarboxyliques;
i) des groupements 2-hydroxyalkylsulfonates;
j) des groupements 2-hydroxyalkylthiosulfate;
k) des groupements hydroxyacylamino.

13. Composition selon l'une quelconque des revendications 1 à 8, caractérisée par le fait que la silicone est une silicone ayant un point d'ébullition compris entre 60 et 260°C.

14. Composition selon la revendication 13, caractérisée par le fait que la silicone volatile est choisie parmi les silicones cycliques de 3 à 7, et de préférence de 4 à 5 atomes de carbone, et leurs mélanges avec des composés organiques dérivés du silicium, ou bien des cyclocopolymères de la famille des diméthylsiloxane/méthylalkylsiloxane, les silicones linéaires ayant de 2 à 9 atomes de silicium.

15. Composition selon l'une quelconque des revendications 1 à 14, caractérisée par le fait que le myristate d'alcool polyhydrique est un myristate d'éthylèneglycol, de propylèneglycol ou de glycérol.

16. Composition selon la revendication 15, caractérisée par le fait que l'on utilise le dimyristate d'éthylèneglycol contenant au moins 95% de radicaux en C₁₄.

17. Composition selon l'une quelconque des revendications 1 à 16, caractérisée par le fait que l'alkyléther d'alcanolamide est l'alcoxy(C₁₃-C₁₅) diéthoximéthyl monoéthanolamide.

18. Composition selon l'une quelconque des revendications 1 à 17, caractérisée par le fait que l'alcanolamide d'acide gras en C₈-C₁₆ est choisi parmi le diéthanolamide de coprah, le monoéthanolamide de coprah ou le monoisopropanolamide de coprah.

19. Composition de lavage et de conditionnement des matières kératiniques, selon la revendication 1 caractérisée par le fait qu'elle contient dans un milieu aqueux :
- de 8 à 30% d'un agent tensio-actif détergent;
- de 1 à 10% et en particulier de 1 à 4% d'une silicone insoluble;
- de 1,5 à 5% de myristate d'un alcool polyhydrique en C₂-C₄;
- de 0,5 à 8% d'un amide choisi parmi les alkyléther d'alcanol(C₁-C₄)amide, le radical alkyle comportant 13 à 15 atomes de carbone et les alcanol (C₁-C₄)amides d'acides gras en C₈-C₁₆.

20. Composition de lavage et de conditionnement des matières kératiniques, selon la revendication 1 caractérisée par le fait qu'elle contient dans un milieu aqueux :
- de 0,1 à 20% en poids d'une silicone insoluble;
- de 8 à 30% en poids d'un agent tensio-actif détergent;
- de 0,1 à 20% de silicone insoluble;
- de 1 à 10% en poids d'acyl (C₇-C₁₇) iséthionate ou d'alkylméthyltaurate;
- de 1 à 8% en poids de myristate d'alcool polyhydrique en C₂-C₄;
- de 0,5 à 8% en poids d'alkyléther d'alcanolamide ou d'alcanolamide d'acide gras en C₈-C₁₆.

21. Composition selon l'une quelconque des revendications 1 à 20, caractérisée par le fait que le pH des compositions est compris entre 3 et 9, et de préférence entre 4 et 8.

22. Composition selon l'une quelconque des revendications 1 à 21, caractérisée par le fait qu'elle contient en outre un agent régulateur de viscosité choisi parmi les électrolytes, les hydrotropes et d'autres épaississants pouvant être présents jusqu'à 10% en poids par rapport au poids total de la composition, des agents nacrants ou opacifiants choisis parmi les palmitates de sodium ou de magnésium, les mono- ou distéarates de l'éthylèneglycol; des agents tensio-actifs cationiques, les polymères anioniques, non-ioniques, cationiques ou amphotères ou des protéines éventuellement quaternisées, ainsi que leurs mélanges.

23. Utilisation comme shampooing de la composition telle que définie dans l'une quelconque des revendications 1 à 22.

24. Utilisation comme gel douche de la composition définie dans l'une quelconque des revendications 1 à 22.

25. Procédé de lavage et de conditionnement des cheveux, caractérisé par le fait que l'on applique sur les cheveux au moins une composition telle que définie dans l'une quelconque des revendications 1 à 22 et qu'après un temps de pose, on procède au rinçage et éventuellement à un nouveau lavage.

## Patentansprüche

1. Wasch- und Konditionierzusammensetzung für keratinhaltiges Material, insbesondere für Haare und Haut, dadurch
**gekennzeichnet,** daß sie in einem wäßrigen Medium enthält:
- mindestens ein oberflächenaktives Detergens,
- mindestens ein unlösliches Silicon,
- mindestens ein Myristat eines mehrwertigen C₂-C₄ Alkohols,
- mindestens einen Alkylether eines C₁-C₄ Alkanolamids und/oder ein C₁-C₄ Alkanolamid einer C₈-C₁₆ Fettsäure.

2. Zusammensetzung gemäß Anspruch 1, dadurch **gekennzeichnet**, daß sie enthält:
- 5 bis 50 Gew.-% mindestens eines oberflächenaktiven Detergens,
- 0,1 bis 20 Gew.-% mindestens eines unlöslichen Silicons,
- 1 bis 8 Gew.-% eines Myristats eines mehrwertigen C₂-C₄ Alkohols, und
- 0,5 bis 8 Gew.-% eines Alkanolamidalkylethers und/oder eines C₁-C₄ Alkanolamids einer C₈-C₁₆ Fettsäure.

3. Zusammensetzung gemäß Anspruch 1 oder 2, dadurch **gekennzeichnet**, daß die oberflächenaktiven Detergenzien ausgewählt werden aus anionischen, amphoteren oder zwitterionischen Tensiden, nichtionischen Tensiden oder Mischungen davon.

4. Zusammensetzung gemäß einem der Ansprüche 1 bis 3, dadurch **gekennzeichnet**, daß die anionischen oberflächenaktiven Mittel ausgewählt werden aus Alkalisalzen, Magnesiumsalzen, Ammoniumsalzen, Aminsalzen oder Aminoalkoholsalzen der folgenden Verbindungen:
- Alkylsulfate, Alkylethersulfate, Alkylamidoethersulfate, Alkylarylpolyethersulfate, Monoglyceridsulfate;
- Alkylsulfonate, Alkylamidsulfonate, Alkylarylsulfonate, Olefinsulfonate, Paraffinsulfonate;
- Alkylsulfosuccinate, Alkylethersulfosuccinate, Alkylamid-sulfosuccinate;
- Alkylsulfosuccinamate;
- Alkylsulfoacetate;
- Alkylphosphate, Alkyletherphosphate;
- Acylsarcosinate;
- wobei die Alkyl- oder Acylgruppe dieser Verbindungen aus einer Kohlenstoffkette mit 12 bis 20 Kohlenstoffatomen besteht;
- Salze von Fettsäuren, ausgewählt aus Salzen der Ölsäure, Ricinolsäure, Palmitinsäure, Stearinsäure; Säuren aus Kopraöl oder hydriertem Kopraöl; Acyllactylate, wobei das Acylradikal 8 bis 20 Kohlenstoffatome hat;
- Säuren von polyoxyalkylenierten Carboxyethern der Formel:
R-(OC₃H₆)ₚ-(OC₂H₄)ₙ-OCH₂COOA
worin R eine Alkylgruppe oder eine Mischung aus C₈-C₂₂-linearen oder verzweigten Alkyl- oder Alkenylgruppen, eine (C₈-C₉)Alkylphenylgruppe, eine Gruppe
R′CONH-CH₂-CH₂-, wobei R′ ein lineares oder verzweigtes C₁₁-C₂₁ Alkyl- oder Alkenylradikal bezeichnet, darstellt;
n eine Ganzzahl oder Dezimalzahl zwischen 2 und 24 ist,
p eine Ganzzahl oder Dezimalzahl zwischen 0 und 6 ist,
A ein Wasserstoffatom oder Na, K, Li, 1/2 Mg oder einen Monoethanolamin-, Ammonium- oder Triethanolaminrest bezeichnet.

5. Zusammensetzung gemäß einem der Ansprüche 1 bis 4, dadurch **gekennzeichnet**, daß das oberflächenaktive Mittel ein nichtionisches Tensid ist, das ausgewählt ist aus:
- polyethoxylierten, polypropoxylierten, polyglycerinisierten Alkoholen, Alkylphenolen und Fettsäuren mit einer linearen Fettsäurekette mit 8 bis 18 Kohlenstoffatomen, wobei die Zahl der Ethylenoxidgruppen und Propylenoxidgruppen zwischen 2 und 50 liegt und die Zahl der Glyceringruppen zwischen 2 und 30 liegt;
- Copolymere von Ethylenoxid und Propylenoxid;
- Kondensate von Ethylenoxid und Propylenoxid mit Fettalkoholen;
- polyethoxylierten Fettsäureamiden;
- polyglycerinisierten Fettsäuresamiden;
- polyethoxylierten Fettaminen;
- oxyethylenierten Sorbitanfettsäureestern;
- Sucrosefettsäureestern;
- Polyethylenglycolfettsäureestern;
- Phosphorsäuretriestern;
- Aminoxiden.

6. Zusammensetzung gemäß einem der Ansprüche 1 bis 5, dadurch **gekennzeichnet,** daß die amphoteren oder zwitterionischen oberflächenaktiven Mittel ausgewählt werden aus:
- Derivaten von sekundären oder tertiären alphatischen Aminen, bei denen die aliphatische Gruppe eine lineare oder verzweigte Kette mit 8 bis 18 Kohlenstoffatomen ist, die mindestens eine anionische wasserlösliche Carboxyl-, Sulfonat-, Sulfat-, Phosphat oder Phosphonatgruppe enthält;
- C₈-C₂₀ Alkylbetainen, C₈-C₂₀-Alkylsulfobetainen, C₈-C₂₀-Alkylamido(C₁-C₆)alkylbetainen oder C₈-C₂₀ Alkylamido(C₁-C₆)alkylsulfobetainen.

7. Zusammensetzung gemäß einem der Ansprüche 1 bis 6, dadurch **gekennzeichnet,** daß man Mischungen aus oberflächenaktiven Detergenzien und insbesondere Mischungen aus anionischen Tensiden und amphoteren, zwitterionischen oder nichtionischen Tensiden verwendet.

8. Zusammensetzung gemäß einem der Ansprüche 1 bis 7, dadurch **gekennzeichnet,** daß das Silicon ausgewählt ist aus Polyorganosiloxanen, die in wäßrigen Medien unlöslich sind und in Form von Ölen, Wachsen, Gummis oder Harzen vorliegen.

9. Zusammensetzung gemäß einem der Ansprüche 1 bis 8, dadurch **gekennzeichnet**, daß das Silicon ein nichtflüchtiges Silicon ist, das ausgewählt ist aus Polyalkylsiloxanen, Polyarylsiloxanen, Polyalkylarylsiloxanen, Silicongummis oder Harzen, organomodifizierten Polysiloxanen und Mischungen davon.

10. Zusammensetzung gemäß Anspruch 9, dadurch **gekennzeichnet,** daß das nichtflüchtige Silicon ausgewählt ist aus:
- linearen Polydimethylsiloxanen mit Trimethylsilyl-Endgruppen,
- linearen Polydimethylsiloxanen mit Dimethylsilanol-Endgruppen,
- C₁-C₂₀ Polylalkylsiloxanen mit mindestens einer C₁-C₂₀ Fettsäurekette,
- linearen und/oder verzweigten Polydimethylmethylphenylsiloxanen, Polydimethyldiphenylsiloxanen,
- Silicongummis mit Molmassen zwischen 200.000 und 1.000.000, die einzeln oder in Form von Mischungen in einem Lösungsmittel verwendet werden,
- Organopolysiloxanharzen, die aus vernetzten Siloxansystemen bestehen und die Einheiten R₂SiO₂/2, RSiO₃/2 und SiO₄/2 umfassen, worin R eine Kohlenwasserstoffgruppe mit 1 bis 6 Kohlenstoffatomen oder eine Phenylgruppe darstellt;
- organomodifizierten Siliconen, ausgewählt aus Siliconen, die in ihrer Struktur eine oder mehrere organofunktionelle Gruppen enthalten, die direkt an die Siloxankette oder dazwischen an eine Kohlenwasserstoffgruppe gebunden sind.

11. Zusammensetzung gemäß Anspruch 10,
dadurch **gekennzeichnet**, daß die Silicongummis ausgewählt sind aus der Gruppe bestehend aus den folgenden Copolymeren:
- Poly/(dimethylsiloxan)/(methylvinylsiloxan)/,
- Poly/(dimethylsiloxan)/(diphenylsiloxan)/,
- Poly/(dimethylsiloxan)/(phenylmethylsiloxan)/,
- Poly/(dimethylsiloxan)/(diphenylsiloxan)/(methylvinylsiloxan)/;
und den folgenden Mischungen:
- Mischungen, die aus einem Polydimethylsiloxan, das an den Kettenenden hydroxyliert ist, und aus einem zyklischen Polydimethylsiloxan gebildet werden;
- Mischungen, die aus einem Polydimethylsiloxangummi und einem zyklischen Silicon gebildet werden;
- Mischungen aus zwei Polydimethylsiloxanen mit verschiedener Viskosität.

12. Zusammensetzung gemäß Anspruch 10,
dadurch **gekennzeichnet,** daß die organomodifizierten Silicone ausgewählt werden aus Polyorganosiloxanen, welche enthalten:
a) Polyethylenoxy- und/oder Polypropylenoxygruppen, die ggf. Alkylgruppen tragen;
b) substituierten oder nichtsubstituierten Aminogruppen;
c) Thiolgruppen;
d) Carboxylatgruppen;
e) Alkoxygruppen;
f) Hydroxyalkylgruppen der folgenden Formel 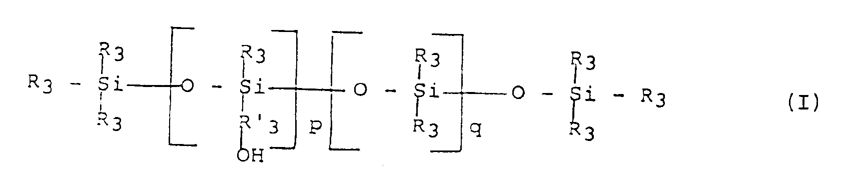 worin die Gruppen R₃, die gleich oder verschieden sind, ausgewählt werden aus Methyl- und Phenylgruppen, wobei mindestens 60 Mol-% der R₃-Radikale Methyl sind;
das Radikal R′₃ eine zweiwertige C₂-C₁₈-Alkylenkohlenwasserstoffkette ist;
p zwischen 1 und einschließlich 30 liegt;
q zwischen 1 und einschließlich 150 liegt;
g) Acyloxyalkylgruppen der folgenden Formel (II): 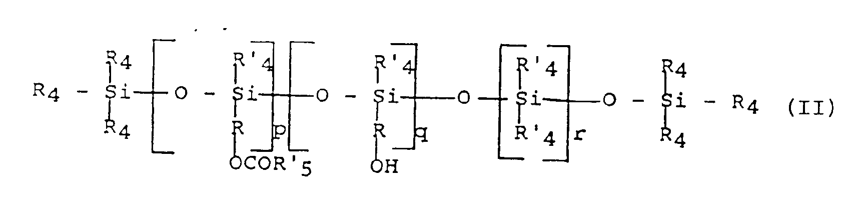 worin:
R₄ Methyl, Phenyl, -OCOR˝, oder Hydroxy bezeichnet und ein R₄ pro Siliciumatom OH sein kann;
R′₄ Methyl oder Phenyl bezeichnet, wobei mindestens 60 Mol-% der beiden Gruppen R₄ und R′₄ zusammengenommen Methyl sind;
R₅ C₈-C₂₀-Alkyl oder -Alkenyl bezeichnet;
R einen zweiwertigen linearen oder verzweigten C₂-C₁₈ Alkylenkohlenwasserstoff bezeichnet;
r zwischen 1 und einschließlich 120 liegt;
p zwischen 1 und 30 liegt;
q gleich 0 oder kleiner als 0,5 p ist, wobei die Summe p+q zwischen 1 und 30 liegt; wobei die Polyorganosiloxane der Formel (II) 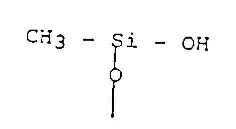 Gruppen enthalten können, deren Anteil 15 % der Summe von p+q+r nicht übersteigt;
h) Alkylcarboxylgruppen;
i) 2-Hydroxyalkylsulfonatgruppen;
j) 2-Hydroxyalkylthiosulfatgruppen und
k) Hydroxyacylaminogruppen.

13. Zusammensetzung gemäß einem der Ansprüche 1 bis 8, dadurch **gekennzeichnet,** daß das Silicon einem Siedepunkt zwischen 60 und 260 °C hat.

14. Zusammensetzung gemäß Anspruch 13, dadurch **gekennzeichnet,** daß das flüchtige Silicon ausgewählt wird aus zyklischen Siliconen mit 3 bis 7 und vorzugsweise 4 bis 5 Kohlenstoffatomen und Mischungen davon mit siliciumorganischen Verbindungen oder aus Cyclocopolymeren aus der Familie der Dimethylsiloxane/Methylalkylsiloxane, wobei die linearen Silicone 2 bis 9 Siliciumatome aufweisen.

15. Zusammensetzung gemäß einem der Ansprüche 1 bis 14, dadurch **gekennzeichnet,** daß das Myristat des mehrwertigen Alkohols ein Myristat des Ethylenglycols, Propylenglycols oder von Glycerin ist.

16. Zusammensetzung gemäß Anspruch 15, dadurch **gekennzeichnet,** daß man Ethylenglycoldimyristat, das mindestens 95 % C₁₄-Gruppen enthält, verwendet.

17. Zusammensetzung gemäß einem der Ansprüche 1 bis 16, dadurch **gekennzeichnet,** daß der Alkanolamidalkylether C₁₃-C₁₅ Alkoxydiethoxymethylmonoethanolamid ist.

18. Zusammensetzung gemäß einem der Ansprüche 1 bis 17, dadurch **gekennzeichnet**, daß das Alkanolamid der C₈-C₁₆ Fettsäure ausgewählt wird aus Kopradiethanolamid, Kopramonoethanolamid oder Kopramonoisopropanolamid.

19. Wasch- und Konditionierzusammensetzung für keratinhaltiges Material gemäß Anspruch 1, dadurch **gekennzeichnet,** daß sie in einem wäßrigen Medium enthält:
- 8 bis 30 % eines oberflächenaktiven Detergens;
- 1 bis 10 % und insbesondere 1 bis 4 % eines unlöslichen Silicons;
- 1,5 bis 5 % eines Myristats eines mehrwertigen C₂-C₄ Alkohols;
- 0,5 bis 8 % eines Amids, ausgewählt aus Alkylethern eines C₁-C₄-Alkanolamids, wobei die Alkylgruppe 13 bis 15 Kohlenstoffatome enthält, und C₁-C₄-Alkanolamiden von C₈-C₁₆-Fettsäuren.

20. Wasch- und Konditionierzusammensetzung für keratinhaltiges Material gemäß Anspruch 1, dadurch **gekennzeichnet,** daß sie in einem wäßrigen Medium enthält:
- 0,1 bis 20 Gew.-% eines unlöslichen Silicons;
- 8 bis 30 Gew.-% eines oberflächenaktiven Detergens;
- 0,1 bis 20 % unlösliches Silicon;
- 1 bis 10 Gew.-% C₇-C₁₇-Acylisethionat oder Alkylmethyltaurat;
- 1 bis 8 Gew.-% Myristat eines mehrwertigen C₂-C₄ Alkohols;
- 0,5 bis 8 Gew.-% eines Alkanolamidalkylethers oder eines Alkanolamids einer C₈-C₁₆ Fettsäure.

21. Zusammensetzung gemäß einem der Ansprüche 1 bis 20, dadurch **gekennzeichnet,** daß der pH der Zusammensetzungen zwischen 3 und 9 und vorzugsweise zwischen 4 und 8 liegt.

22. Zusammensetzung gemäß einem der Ansprüche 1 bis 21, dadurch **gekennzeichnet,** daß sie außerdem ein viskositätsregulierendes Mittel enthält, ausgewählt aus Elektrolyten, Hydrotropen und anderen Verdickern, die in Mengen von bis zu 10 Gew.-%, bezogen auf die Gesamtzusammensetzung, vorliegen können, perlmuttglanzverleihenden oder opalizierenden Mitteln, ausgewählt aus Natrium- oder Magnesiumpalmitat, Ethylenglycolmono- oder Distearat; kationischen oberflächenaktiven Mitteln, anionischen, nichtionischen, kationischen oder amphoteren Polymeren oder ggf. quaternisierten Proteinen, und Mischungen davon.

23. Verwendung einer Zusammensetzung gemäß einem der Ansprüche 1 bis 22 als Shampoo.

24. Verwendung einer Zusammensetzung gemäß einem der Ansprüche 1 bis 22 als Duschgel.

25. Verfahren zum Waschen und Konditionieren von Haaren, dadurch **gekennzeichnet,** daß man auf die Haare mindestens eine Zusammensetzung gemäß einem der Ansprüche 1 bis 22 aufbringt und nach einer Ruhezeit zum Auswaschen und ggf. einer neuen Wäsche übergeht.

## Claims

1. Composition for washing and conditioning keratinous substances, in particular hair and the skin, characterised in that it contains in an aqueous medium
- at least one detergent surface-active agent,
- at least one insoluble silicone,
- at least one myristate of a C₂-C₄ polyhydric alcohol,
- at least one (C₁-C₄)alkanolamide alkyl ether and/or C₈-C₁₆ fatty acid (C₁-C₄) alkanolamide.

2. Composition according to Claim 1, characterised in that it contains:
- from 5 to 50 % by weight of at least one detergent surface-active agent,
- from 0.1 to 20 % by weight of at least one insoluble silicone,
- from 1 to 8 % by weight of myristate of a C₂-C₄ polyhydric alcohol, and
- from 0.5 to 8 % by weight of alkanolamide alkyl ether and/or of C₈-C₁₆ fatty acid (C₁-C₄)alkanolamide.

3. Composition according to Claim 1 or 2, characterised in that the detergent surface-active agents are chosen from anionic, amphoteric or zwitterionic or nonionic surface-active agents or their mixtures.

4. Composition according to any one of Claims 1 to 3, characterised in that the anionic surface-active agents are chosen from the alkali metal salts, the magnesium salts, the ammonium salts, the amine salts or the aminoalcohol salts of the following compounds:
- alkyl sulphates, alkyl ether sulphates, alkylamidoether sulphates, alkylaryl polyether sulphates and monoglyceride sulphates;
- alkylsulphonates, alkylamidesulphonates, alkylarylsulphonates, olefin sulphonates, paraffin sulphonates;
- alkylsulphosuccinates, alkyl ether sulphosuccinates, alkylamidesulphosuccinates;
- alkylsulphosuccinamates;
- alkylsulphoacetates;
- alkyl phosphates, alkyl ether phosphates;
- acylsarcosinates; the alkyl or acyl radical in these compounds consisting of a carbon chain containing 12 to 20 carbon atoms;
- fatty acid salts chosen from the salts of oleic, ricinoleic, palmitic and stearic acids; copra oil or hydrogenated copra oil acids, and acyllactylates in which the acyl radical contains from 8 to 20 carbon atoms;
- polyoxyalkylenated carboxylic ether acids of formula:
R-(OC₃H₆)ₚ-(OC₂H₄)ₙ-OCH₂COOA
in which R denotes an alkyl radical or a mixture of linear or branched, C₈-C₂₂ alkyl or alkenyl radicals, a (C₈-C₉)alkylphenyl radical, an R′CONH-CH₂-CH₂- group with R′ denoting a C₁₁-C₂₁ linear or branched alkyl or alkenyl radical;
n is an integer or decimal number between 2 and 24,
p is an integer or decimal number between 0 and 6,
A denotes a hydrogen atom or Na, K, Li, ½ Mg or a monoethanolamine, ammonium or triethanolamine residue.

5. Composition according to any one of Claims 1 to 4, characterised in that the surface-active agent is a nonionic surface-active agent chosen from:
- alcohols, alkylphenols and polyethoxylated, polypropoxylated or polyglycerolated fatty acids containing a linear fatty chain containing 8 to 18 carbon atoms, the number of ethylene oxide or propylene oxide groups being between 2 and 50 and the number of glycerol groups being between 2 and 30;
- copolymers of propylene and ethylene oxide;
- condensates of propylene and ethylene oxide with fatty alcohols;
- polyethoxylated fatty amides;
- polyglycerolated fatty amides;
- polyethoxylated fatty amines;
- oxyethylenated sorbitan fatty acid esters;
- sucrose fatty acid esters;
- polyethylene glycol fatty acid esters;
- phosphoric triesters;
- amine oxides.

6. Composition according to any one of Claims 1 to 5, characterised in that the amphoteric or zwitterionic surface-active agents are chosen from:
- derivatives of aliphatic secondary or tertiary amines in which the aliphatic radical is a linear or branched chain containing 8 to 18 carbon atoms and which contains at least one carboxylic, sulphonate, sulphate, phosphate or phosphonate hydrosolubilising anionic group;
- (C₈-C₂₀)alkylbetaines, (C₈-C₂₀)alkylsulphobetaines, (C₈-C₂₀)alkylamido(C₁-C₆)alkylbetaines or (C₈-C₂₀)alkylamido(C₁-C₆)alkylsulphobetaines.

7. Composition according to any one of Claims 1 to 6, characterised in that mixtures of detergent surface-active agents and in particular mixtures of anionic surface-active agents and of amphoteric, zwitterionic or nonionic surface-active agents are employed.

8. Composition according to any one of Claims 1 to 7, characterised in that the silicone is chosen from polyorganosiloxanes which are insoluble in aqueous media, which are in the form of oils, waxes, gums or resins.

9. Composition according to any one of Claims 1 to 8, characterised in that the silicone is a nonvolatile silicone chosen from polyalkylsiloxanes, polyarylsiloxanes, polyalkylarylsiloxanes, silicone gums and resins, organomodified polysiloxanes and mixtures thereof.

10. Composition according to Claim 9, characterised in that the nonvolatile silicone is chosen from:
- linear polydimethylsiloxanes containing trimethylsilyl end groups,
- linear polydimethylsiloxanes containing dimethylsilanol end groups,
- poly(C₁-C₂₀)alkylsiloxanes containing at least one C₁-C₂₀ fatty chain,
- linear and/or branched polydimethylmethylphenylsiloxanes and polydimethyldiphenylsiloxanes,
- silicone gums of molecular mass of between 200,000 and 1,000,000, employed by themselves or in the form of mixtures in a solvent,
- organopolysiloxane resins consisting of crosslinked siloxane systems containing the units R₂SiO_{2/2}, RSiO_{3/2} and SiO_{4/2}, in which R denotes a hydrocarbon group containing from 1 to 6 carbon atoms or a phenyl group;
- organomodified silicones chosen from silicones containing in their structure one or more organofunctional groups attached directly to the siloxane chain or attached by means of a hydrocarbon radical.

11. Composition according to Claim 10, characterised in that the silicone gums are chosen from the group consisting of the following copolymers:
- poly/(dimethylsiloxane)/(methylvinylsiloxane)/,
- poly/(dimethylsiloxane)/(diphenylsiloxane)/,
- poly/(dimethylsiloxane)/(phenylmethylsiloxane)/,
- poly/(dimethylsiloxane)/(diphenylsiloxane)/(methylvinylsiloxane)/;
and the following mixtures:
- the mixtures made up of a polydimethylsiloxane hydroxylated at a chain end and of a cyclic polydimethylsiloxane;
- the mixtures made up of a polydimethylsiloxane gum and of a cyclic silicone;
- mixtures of two polydimethylsiloxanes of different viscosities.

12. Composition according to Claim 10, characterised in that the organomodified silicones are chosen from polyorganosiloxanes containing:
a) polyethyleneoxy and/or polypropyleneoxy groups, optionally containing alkyl groups;
b) substituted or unsubstituted amine groups;
c) thiol groups;
d) carboxylate groups;
e) alkoxylated groups;
f) hydroxyalkyl groups corresponding to the following formula: 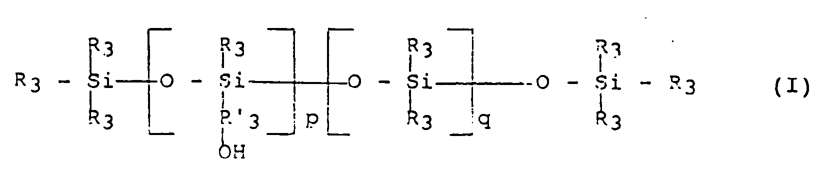 in which:
- the radicals R₃, which are identical or different, are chosen from methyl and phenyl radicals, at least 60 mol% of the radicals R₃ being methyl;
- the radical R′₃ is a C₂-C₁₈ divalent hydrocarbon alkylene chain unit;
- p is between 1 and 30 inclusive;
- q is between 1 and 150 inclusive.
g) acyloxyalkyl groups corresponding to the following formula: 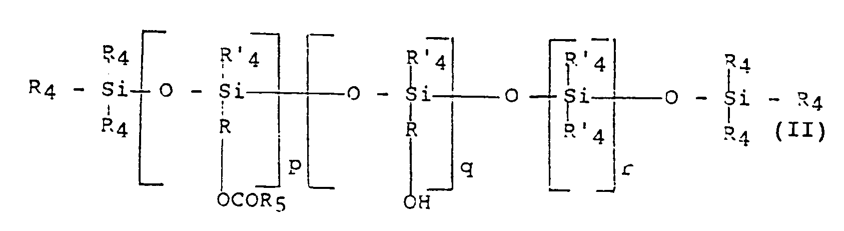 in which:
- R₄ denotes methyl, phenyl, -OCOR˝, hydroxyl, where only one R₄ per silicon atom may be OH;
- R′₄ denotes methyl or phenyl, at least 60 mol% of the total of the radicals R₄ and R′₄ is methyl;
- R₅ denotes C₈-C₂₀ alkyl or alkenyl;
- R denotes a C₂-C₁₈ linear or branched divalent hydrocarbon alkylene;
- r is between 1 and 120 inclusive;
- p is between 1 and 30;
- q is equal to 0 or is smaller than 0.5 p, the sum p+q being between 1 and 30; it being possible for the polyorganosiloxanes of formula (II) to contain 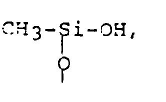 groups,
in proportions not exceeding 15 % of the sum p+q+r;
h) alkylcarboxylic groups;
i) 2-hydroxyalkylsulphonate groups;
j) 2-hydroxyalkylthiosulphate groups;
k) hydroxyacylamino groups.

13. Composition according to any one of Claims 1 to 8, characterised in that the silicone is a volatile silicone which has a boiling point of between 60 and 260°C.

14. Composition according to Claim 13, characterised in that the volatile silicone is chosen from cyclic silicones with 3 to 7 and preferably 4 to 5 carbon [sic] atoms and their mixtures with organic compounds derived from silicon, or cyclocopolymers from the class of dimethylsiloxane/methylalkylsiloxanes, the linear silicones containing from 2 to 9 silicon atoms.

15. Composition according to any one of Claims 1 to 14, characterised in that the polyhydric alcohol myristate is an ethylene glycol, propylene glycol or glycerol myristate.

16. Composition according to Claim 15, characterised in that ethylene glycol dimyristate containing at least 95 % of C₁₄ radicals is employed.

17. Composition according to any one of Claims 1 to 16, characterised in that the alkanolamide alkyl ether is (C₁₃-C₁₅)alkoxydiethoxymethylmonoethanolamide.

18. Composition according to any one of Claims 1 to 17, characterised in that the C₈-C₁₆ fatty acid alkanolamide is chosen from copra diethanolamide, copra monoethanolamide or copra monoisopropanolamide.

19. Composition for washing and conditioning keratinous substances, characterised in that it contains in an aqueous medium:
- from 8 to 30 % of a detergent surface-active agent;
- from 1 to 10 % and in particular from 1 to 4 % of an insoluble silicone;
- from 1.5 to 5 % of myristate of a C₂-C₄ polyhydric alcohol;
- from 0.5 to 8 % of an amide chosen from (C₁-C₄)alkanolamide alkyl ether, the alkyl radical containing 13 to 15 carbon atoms and C₈-C₁₆ fatty acid (C₁-C₄)alkanolamides.

20. Composition for washing and conditioning keratinous substances, characterised in that it contains in an aqueous medium:
- from 0.1 to 20 % by weight of an insoluble silicone;
- from 8 to 30 % by weight of a detergent surface-active agent;
- from 0.1 to 20 % of insoluble silicone;
- from 1 to 10 % by weight of (C₇-C₁₇)acylisethionate or of alkyl methyltaurate;
- from 1 to 8 % by weight of C₂-C₄ polyhydric alcohol myristate;
- from 0.5 to 8 % by weight of alkanolamide alkyl ether or of C₈-C₁₆ fatty acid alkanolamide.

21. Composition according to any one of Claims 1 to 20, characterised in that the pH of the compositions is between 3 and 9 and preferably between 4 and 8.

22. Composition according to any one of Claims 1 to 21, characterised in that it additionally contains a viscosity-regulating agent chosen from electrolytes, hydrotropes and other thickeners which may be present as up to 10 % by weight relative to the total weight of the composition, opalescent or opacifying agents chosen from sodium or magnesium palmitates and ethylene glycol mono- or distearates; cationic surface-active agents, anionic, nonionic, cationic or amphoteric polymers or optionally quaternised proteins and mixtures thereof.

23. Use as a shampoo of the composition as defined in any one of Claims 1 to 22.

24. Use as a shower gel of the composition defined in any one of Claims 1 to 22.

25. Process for washing and conditioning hair, characterised in that at least one composition as defined in any one of Claims 1 to 22 is applied to hair and that after a period of exposure, rinsing and optionally a second washing is carried out.
